Europäisches Patentamt

European Patent Office

Office européen des brevets

(11) Publication number: **0 250 693 B1**

# EUROPEAN PATENT SPECIFICATION

(45) Date of publication of patent specification: **22.04.92** (51) Int. Cl.⁵: **A61B 5/14**, A61B 19/02

(21) Application number: **86830177.1**

(22) Date of filing: **20.06.86**

(54) **Portable device for the instantaneous drawing of blood.**

(43) Date of publication of application:
**07.01.88 Bulletin 88/01**

(45) Publication of the grant of the patent:
**22.04.92 Bulletin 92/17**

(84) Designated Contracting States:
**AT BE CH DE FR GB LI LU NL SE**

(56) References cited:
**EP-A- 0 068 864**
**EP-A- 0 115 388**
**DE-B- 1 079 275**
**FR-A- 2 508 305**

(73) Proprietor: **A. Menarini S.a.S.**
**Via dei Sette Santi 3**
**I-50131 Firenze(IT)**

(72) Inventor: **Basagni, Umberto**
**a. Menarini S.a.s. Via Sette Santi 3**
**I-50131 Firenze(IT)**
Inventor: **Bonicolini, Francesco**
**a. Menarini S.a.s. Via Sette Santi 3**
**I-50131 Firenze(IT)**

(74) Representative: **de Simone, Domenico et al**
**Ing. Barzanò & Zanardo Roma S.p.A. Via**
**Piemonte 26**
**I-00187 Roma(IT)**

## Description

The present invention relates to a device for the instantaneous drawing of blood.

More particularly, the present invention relates to a device of the type set forth above, said device being particularly suitable for drawing blood samples from a finger, in which device a reservoir is provided for housing needles to be used once only, said device giving in addition the possibility of differentiating the drawing depths through the substitution of said closing finger-protecting member for other members having suitable thicknesses according to the user.

Devices designed for the same object as the device of the present invention are already commercially available from some time, said devices having a blood drawing mechanism which is substantially similar to that of the present invention. However, all devices commercially available at the present time show a number of practical drawbacks which make their employment not completely advantageous.

Indeed, more particularly, such kind of drawing is performed in patients suffering from diabetes, in which patients it is necessary to carry out a number of drawing operations on a same day. Very often the patients perform the drawing for themselves independently.

Thus it is clear that, when the patient is far from home for a long time, he has to carry with himself a plurality of needles to be used once only slipping them in his pocket, or in his bag, and so on, running the risk of losing them or that the needles lose their protective member and consequently their sterilization.

Moreover, the penetration depth is to be necessarily differentiated from patient to patient. Indeed, the needle driven by its mechanism will penetrate much more easily into the finger of a child than into the finger of an adult whose hands may show some callosities.

Such differentiation cannot be obtained with devices commercially available at the present time, in which one only type is provided of closing finger-protecting member that is therefore not suitable for all patients.

Devices having the above features are described in EP-A-0,115,388 and 0,068,864, in FR-A-2,508,305 and in DE-B-1,079,275.

In particular, the EP-A-0,115,338 and the DE-B-1,079,275 describe a device comprising a release mechanism, at whose outer and a finger-pricking needle is provided to be used once only, said needle being housed within a containing chamber.

In the device of DE-B-1,079,275 a reservoir for housing a plurality of spare needles, by which said needles are automatically fed, is provided.

In said EP-A-0,115,388 a closing finger-member bearing a hole at a point corresponding to said needle and being interchangeable for differentiating the penetration depth of said needle is described, while FR-A-2,508,305 provides a screwable sleeve to adjust the penetration depth.

From the foregoing considerations it is quite evident that a device according to the present invention shows very pratical as it comprises, in addition to the usual release mechanism, also a reservoir whose sizes are the same as those of the chamber housing said mechanism, in which reservoir a plurality of needles, for instance four needles, to be used once only are housed.

Moreover, it is an object of the present invention to provide a series of finger-protecting closing members having different thicknesses suitably designed to meet the needs of each particular patient.

Thus it is a specific object of the present invention to provide a portable device for the instantaneous drawing of bloods, said device comprising a release mechanism at whose outer end a needle to be used once only is provided for drawing the blood sample and housed within a containing chamber, a finger-protecting closing member bearing a hole at a point corresponding to said needle and being interchangeable for differentiating the penetration depth of said needle, characterized in that a reservoir for housing a plurality of spare needles to be used once only is provided having the same size as said chamber and disposed side by side the same chamber, in that said closing finger-protective member is the closing member also of said reservoir and is interchangeable with other finger-protective member having different thicknesses.

According to a particularly preferred embodiment of the device of the present invention, in said reservoir, four needles to be used once only are contained.

Again according to the present invention, said closing interchangeable finger-protective members are provided preferably in number of three, with increasing thickness according to the lower depth needed for drawing blood, said finger-protective members being of different colors so as to distinguish their thicknesses.

Moreover, the device according to the present invention advantageously is provided at its back end with a clip or clasp member for fastening the same to the pocket of the jacket or the like.

The present invention will be disclosed in the following for illustrative but not for limitative purposes according to a preferred embodiment of the same which is illustrated in the Figures of the enclosed drawing wherein:

Figure 1 is a side view of the device according to the present invention;

Figure 2 is a front view of the device according to the present invention with no closing finger-protective member;

Figure 3 is a longitudinal cross section of the device according to the present invention;

Figure 4 is a front view of the closing finger-protective member;

Figure 5 is a side cross section view of the closing finger-protective member of figure 4;

Figure 6 is a side view of the back cap placed vertically of the device according to the present invention; and

Figure 7 is a side view of the back cap, placed horizontally, of the device of the present invention.

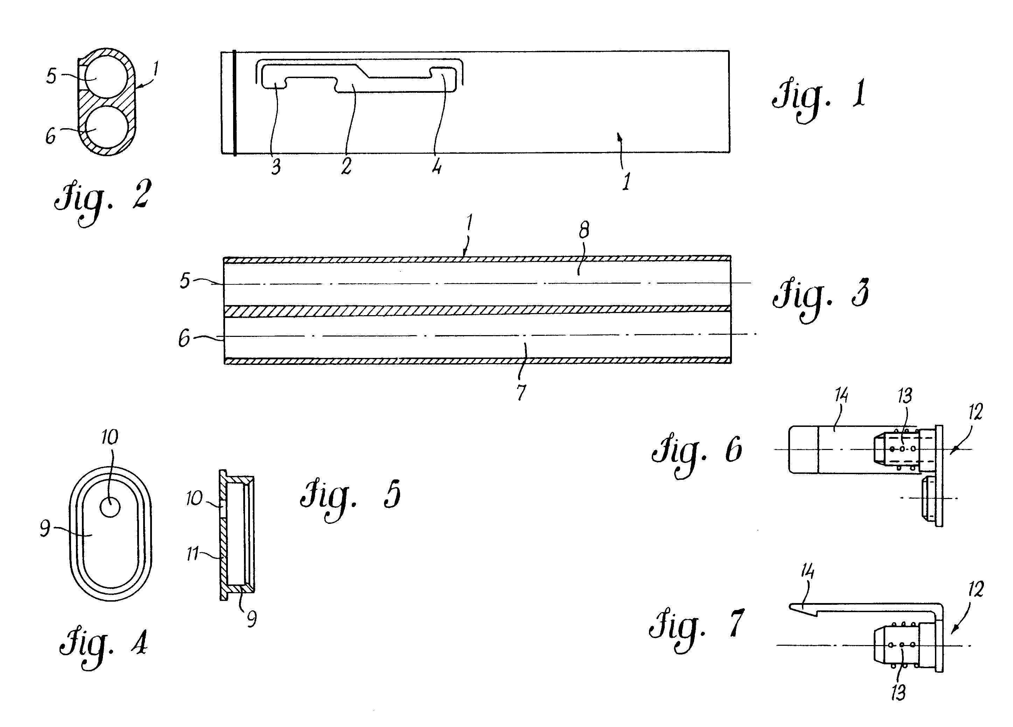

In Figure 1 the reference number 1 points out the device of the present invention provided with a shaped groove 2, for the motion of the pin of the release mechanism (not shown). First said pin is taken to the position pointed out by number 3, where a needle to be used once only is inserted and then loaded in the position 4 so as to press a spring that is not shown.

Figure 2 shows the outlet hole 5 of the finger-pricking needle and the end hole 6 of the reservoir 7 designed for the spare needles (see Figure 3).

Figure 3 also shows the containing chamber 8 of said release mechanism. The closing finger-protective member 9 is provided before said chamber 8 and said reservoir 7 (see Figure 4), said closing member bearing a hole 10 for allowing the needle to go out, said needle being discarded after use. Said hole 10 is clearly provided at a point corresponding to the chamber 8, whereas the part corresponding to the hole 6 of the reservoir 7 is closed.

By changing the tickness 11 of the finger-protective member 9 said needle can be caused to go out to a lower or higher extent, so that the best conditions are realized for drawing blood either from children or from adult patients.

To that aim, the Applicant provides the device with at least three finger-protective members of different thicknesses as well as of different colors so that they can be distinguished more easily.

Indeed, when working, said lever (not shown) is released from the position 4 acting on the pin (not shown) so that the needle, owing to the action exerted by the spring, is pushed through the hole 10 into the finger of the patient.

Figures 6 and 7 illustrate the back cap of the device 1 comprising the locking device of the spring 13. Finally number 14 shows the fastening clip provided on the back of the device, said clip being useful in particular for carrying the device of the present invention when travelling, or when going by bus and so on.

## Claims

1. A portable device (1) for the instantaneous drawing of blood, comprising a release mechanism, at whose outer end a finger-pricking needle is provided to be used once only, said needle being housed within a containing chamber (8), a closing finger-protective member (9) bearing a hole (10) at a point corresponding to said needle and being interchangeable for differentiating the penetration depth of said needle, characterized in that a reservoir (7) for housing a plurality of spare needles to be used once only is provided having the same size as said chamber (8) and disposed side by side the same chamber (8), in that said closing finger-protective member (9) is the closing member also of said reservoir (7) and is interchangeable with other finger-protective members (7) having different thicknesses (11).

2. A portable device (1) for the instantaneous drawing of blood according to claim 1, and device being characterized in that in said reservoir (7), four needles to be used once only are contained.

3. A portable device (1) for the instantaneous drawing of blood according to claims 1 and 2, said device being characterized in that said interchangeable closing finger-protective members (9) are provided at least in number of three, with increasing thickness according to the lower penetration depths required, said finger-protective members (9) being of different colors so as to distinguish the corresponding thickness.

4. A portable device (1) for the instantaneous drawing of blood according to claims 1-3, said device being characterized in that a clip or clasp member (14) is provided at its back end for fastening the device to the jacket and the like.

## Revendications

1. Dispositif portatif de prélèvement instantané de sang, comprenant un mécanisme de relaxation, au bout extérieur duquel est arrangé un aiguille de ponction du doigt utilisable une seule fois, ladite aiguille étant logée dans une chambre d'arrangement (B), un élément de fermeture et de protection du doigt ayant un trou dans un point correspondant à ladite aiguille et étant interchangeable pour différencier la profondeur de pénétration de ladite aiguille, caractérisé en ce qu'un réservoir pour loger une pluralité

d'aiguilles de rechange utilisables une seule fois est arrangé à côté de ladite chambre (B) et à la même dimension; en ce que ledit élément de fermeture et de protection le doigt est l'élément de fermeture aussi dudit réservoir et est interchangeable avec les autres éléments de protection du doigt (7) ayant une épaisseur différente.

2.    Dispositif portatif de prélèvement instantané de sang selon la revendication 1, caractérisé en ce que dans ledit réservoir (7) quatres aiguilles utilisables une seule fois sont placées.

3.    Dispositif portatif de prélèvement instantané de sang, selon les revendications 1 et 2, caracterisé en ce que lesdits éléments interchangeables de fermeture et de protection du doigt (9) sont placés au moins au nombre de trois est ont une épaisseur croissante avec la diminution de la profondité de pénétration requise, lesdits éléments de protection du doigt (9) étant de couleur différente de façon à distinguer l'épaisseur correspondante.

4.    Dispositif portatif de prélèvement instantané du sang selon les revendications 1-3, caractérisé en ce qu'un clip ou un élément d'attache (14) est arrangé sur son bout postérieur pour attacher le dispositif à la veste et pièce semblable.

**Patentansprüche**

1.    Tragbare Vorrichtung (1) zur unmittelbaren Blutentnahme mit einer Freigabe-Vorrichtung, am deren ausseren Ende eine Finger-Stech-Nadel zum nur einmaligen Gebrauch vorgesehen ist, wobei diese Nadel in einern Behaelter (8) untergebracht ist, der mit einem, Fingerschutzglied (9) bedeckt ist, das an einer, der genanten Nadel entsprechenden Stelle mit einem Loch versehen ist und zur Ermoeglidung der unteschiedlichen Einstichtiefen austauschbar ist, dadurch gekennzeicnet, dass zur Aufbewahrung von nur einmal gebrauchbaren Austausch-Nadeln eine, die gleiche Groesse des Behaelters (8) aufweisende und an diesen anliegende Kammer (7) vorgesehen ist, dass dieses Finger-Schutz-Glied (9) auch die Bedeckung der Kammer (7) bildet und durch andere Finger-Schutz-Glieder austauschbar ist, die unterschiellichen Dicken aufweisen.

2.    Tragbare Vorrichtung (1) zur unmittelbaren Blutentnahme nach dem Anspruch 1, dadurch gekennzeidenet, dass in der Aufbewahrunskammer (7) vier nur einmal gebrauchbare Nadeln enthalten sind.

3.    Tragbare Vorrichtung (1) zur unmittelbaren Blutentnahme gemaess den Anspruechen 1 und 2, dadurche gekennzeichnet, dass die genannten austauschbaren Finger-Schutz-Glieder in einer Anzahl von drei vorgesehen sind, eine sich mit der Erniedrigung der notwendigen Einstich-Tiefe vergroessernde Dicke aufweisen und zur Unterscheidung der entsprechenden Dicke eine unterschiedliche Faerbung aufweisen.

4.    Tragbare Vorrichtung (1) zur unmittelbaren Blutentnahme nach den Anspruechen 1 bis 3, dadurch gekennzeichnet, dass an seinem hinteren Ende ein Klipp oder eine Klemmvorrichtung zum Anhaften an die Jacke o. dgl. vorgesehen ist.

Fig. 1

Fig. 2

Fig. 3

Fig. 4

Fig. 5

Fig. 6

Fig. 7